Europäisches Patentamt

**European Patent Office**

Office européen des brevets

(11) Publication number: **0 065 187**
A2

# (12) EUROPEAN PATENT APPLICATION

(21) Application number: **82103831.2**

(22) Date of filing: **05.05.82**

(51) Int. Cl.³: **A 61 B  1/00,** B 65 D  5/70

(30) Priority: **13.05.81  JP 71689/81**

(43) Date of publication of application: **24.11.82**
**Bulletin 82/47**

(84) Designated Contracting States: **AT BE CH DE FR GB IT
LI NL SE**

(71) Applicant: **OLYMPUS OPTICAL CO., LTD.,**
**43-2, 2-chome, Hatagaya Shibuya-ku, Tokyo (JP)**

(72) Inventor: **Ueda, Yasuhiro, 1-22-15, Tokura Kokubunji-shi,**
**Tokyo (JP)**

(74) Representative: **Rüger, Rudolf, Dr.-Ing.,**
**Webergasse 3 Postfach 348, D-7300 Esslingen/Neckar**
**(DE)**

(54) **Plug for closing the forceps inlet of an endoscope.**

(57) An endoscope is provided with a forceps channel (40),
which is communicated with a forceps inlet (38) defined by
a holder (24) on a control section (6). A plug (26) is attached
to the holder (24) to prevent liquid in the body cavity from
leaking outside the forceps inlet (38) through the forceps
channel (40). The plug (26) is cap-shaped and has a first
portion fitted onto the holder (24) and a second portion for
closing the forceps inlet (38). The second portion is made
thinner by forming a recess (54) through which operating
tools such as forceps are penetrated.

ACTORUM AG

**Patentanwälte  Dipl.-Ing. W. Scherrmann  Dr.-Ing. R. Rüger**

7300 Esslingen (Neckar). Webergasse 3. Postfach 348

PA 10 EU hs
May 4, 1982

Telefon
Stuttgart (07 11) 35 65 39
35 96 19
Telex    07 256610 smru
Telegramme Patentschutz
Esslingenneckar

- 1 -

## Plug for closing the forceps
## inlet of an endoscope

The present invention relates to a plug for closing the forceps inlet of an endoscope.

For inserting operating tools such as forceps into a body cavity, the control section of endoscope is provided with a forceps inlet communicated with a forceps channel which extends in the endoscope to open at the distal end thereof. When the insertion section of endoscope is inserted into the body cavity with the forceps inlet opened, there is a fear that air, liquid and dirt in the body cavity leak outside the forceps inlet through the forceps channel. It is therefore necessary to close the forceps inlet with a plug.

The conventional well known plug for closing the forceps inlet is disclosed by Japanese Utility Model Publication No. 26226/72. This plug comprises a pair of resilient covers overlapped one upon the other to close the forceps inlet, and a ring-shaped stopper attached to a holder, which defines the forceps inlet, to fix the pair of covers to the forceps inlet, each of resilient covers being provided with crossed slits. These crossed slits of each of resilient covers prevent liquid and the like in the body cavity from leaking outside the forceps inlet through the forceps channel, but enable forceps to be inserted into the forceps channel through covers.

Resilient covers and plug are different parts in the case of this conventional plug. It is therefore difficult to attach the plug to the holder, which defines the forceps inlet, and to exchange covers fixed in the holder with new ones. In addition, each of covers must be provided with crossed slits and the pair of covers and the stopper must be manufactured separately, thus causing the cost of plug to be made high.

The object of the present invention is to provide a plug for closing the forceps inlet of an endoscope, said plug being simple in construction and easy to manufacture and to exchange with new one.

According to the present invention is provided a cap-shaped plug made of resilient material. The plug has a shape fitted to a member which defines the forceps inlet communicated with a forceps channel extending through the endoscope. That portion of plug through which operating tools such as forceps are inserted is made thinner as compared with the remaining portion thereof.

This invention can be more fully understood from the following detailed description when taken in conjunction with the accompanying drawings, in which:

Fig. 1 is a perspective view showing an endoscope;

Fig. 2 is a plane view showing the distal end of endoscope shown in Fig. 1;

Fig. 3 is a sectional view showing an example of plug according to the present invention and attached to a holder provided in the control section of endoscope shown in Fig. 1;

Fig. 4 is a sectional view showing how operating tools such as forceps are inserted through the plug into the holder shown in Fig. 3;

Figs. 5, 6 and 7 are sectional view showing other examples of plug according to the present invention and attached to the holder;

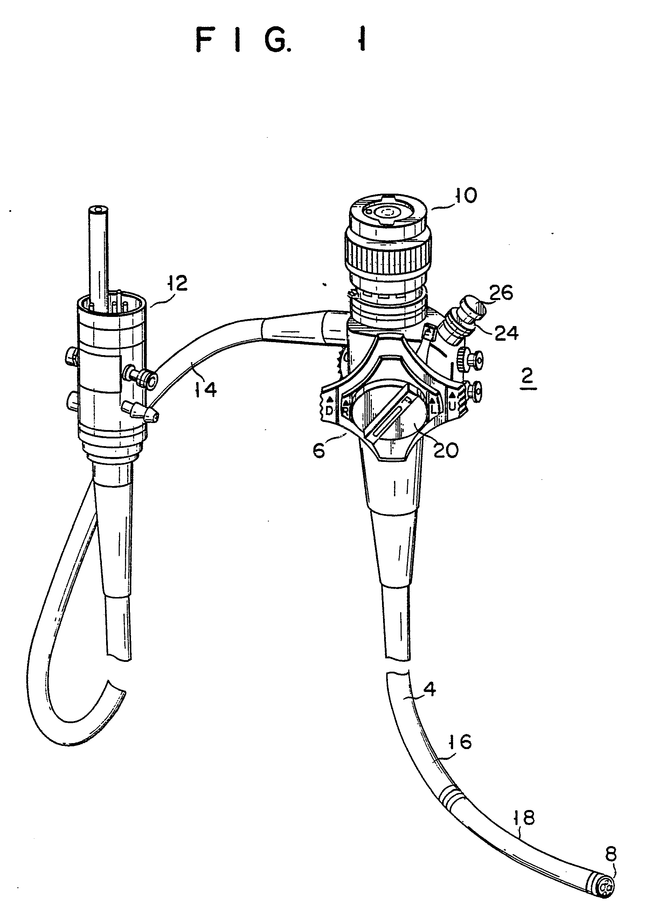

As shown in Fig. 1, an endoscope 2 comprises an insertion section 4 inserted into the body cavity, a control section 6 to which the insertion section 4 is coupled and which serves to control the endoscope 2, an eye piece section 10 for viewing the image of interested region in the body cavity, said image being transmitted through an image guide extending from a distal end 8 of insertion section 4 to the control section 6, and a universal code 14 extending from the control section 6 and provided with a connector to be connected to a light source unit, said universal code houding a light guide therein, which further extends from the control section 6 to the distal end of insertion section 4. The control section is provided with an angle operating knob 20 for bending a bending portion 18 connected to a flexible tube 16 of insertion section 4. A holder 24 defineing a forceps inlet 38, through which operating tools such as forceps are inserted, projects from the control section 6 and is closed by a plug 26. The forceps inlet 38 is communicated with a forceps channel (not shown) extending through control and insertion sections 6 and 4, and the forceps channel is also communicated with a suction hole 28 formed at the distal end 8 of insertion section 4. In addition to the suction hole 28, the distal end 8 is provided with illumination windows 30 and 32 facing the end faces of light guide, and with a nozzle 34 for air and water supply. The suction hole 28 is connected through the forceps channel to a suction channel, which extends through the universal code 14 to the connector 12. When the connector 12 is connected to a socket of light source unit, the suction channel is connected to a suction unit. The nozzle 34 is connected to an air and water supply channel extending through insertion and control sections 4, 6 and the universal code 14. The air and water supply channel is connected to an air and water supply unit in the light source unit when the connector 12 is connected to the light source unit. Similarly, the light guide is optically connected to the light source unit when the connector 12 is connected to the light source unit. The distal end 8 is also provided with an observation window 36 having an objective lens and facing the end face of image guide.

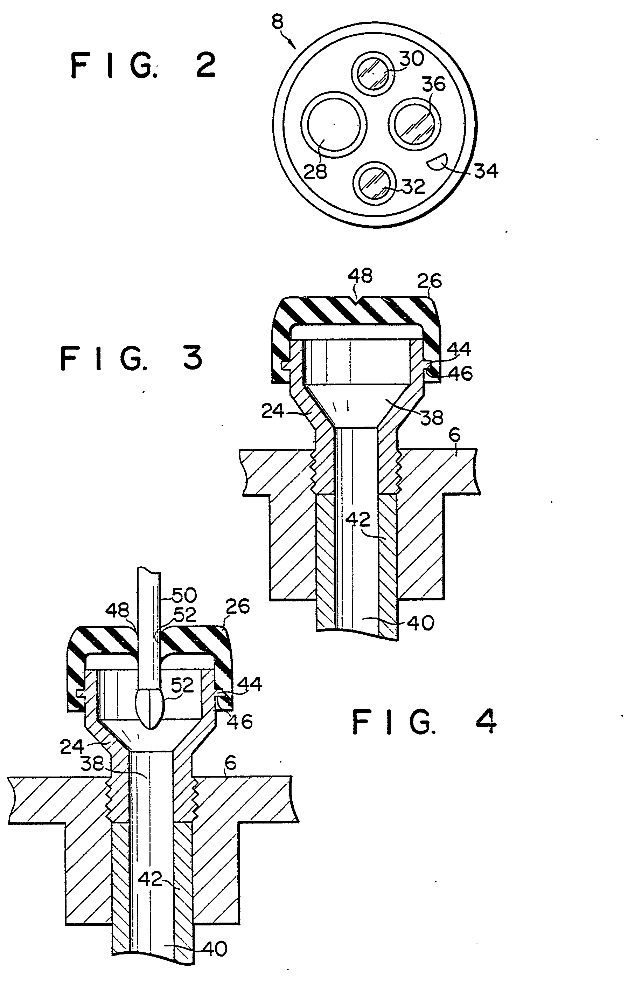

The holder 24 is screwed into the control section 6, as shown in Fig. 3, and its forceps inlet 38 is communicated with a forceps channel 40 formed in the control section and defined by a tube 42. The holder 24 is formed to have a funnel shape, as shown in Fig. 3, and has a projection 44 formed around the outer circumference thereof. The holder 24 is covered by a cap-shaped plug 26, which has a recess 46 formed around the inner circumference thereof. The plug 26 is reliably attached to the holder 24 and the forceps inlet 38 is closed by the plug 26 by fitting the projection 44 of holder 24 into the recess 46 of plug 26. The plug 26 is made of resilient material such as silicon rubber, for example, and a conical recess 48 is formed in the center portion of upper surface of plug 26, so that the center portion thereof is made thinner than the remaining portion thereof.

When no operating tool such as forceps in used, the forceps inlt is liquid-tightly closed by the plug 26, as shown in Fig. 3. When an operating tool 50 such as forceps is used, the foremost end 52 of tool 50 is forced into the recess 48 of plug 26, thus passing through the plug 26 into the forceps inlet 38 and then into the forceps channel 40. The operating tool 50 contacts closely with the inner surface of a through-hole 52 which has been formed by forcing the tool 50 into the plug 26, thus enabling liquid-tightness to be held. When the tool 50 is removed, the through-hole 54 is closed by the restoring force of resilient plug 26, thus enabling the forceps inlet 38 to be sealed.

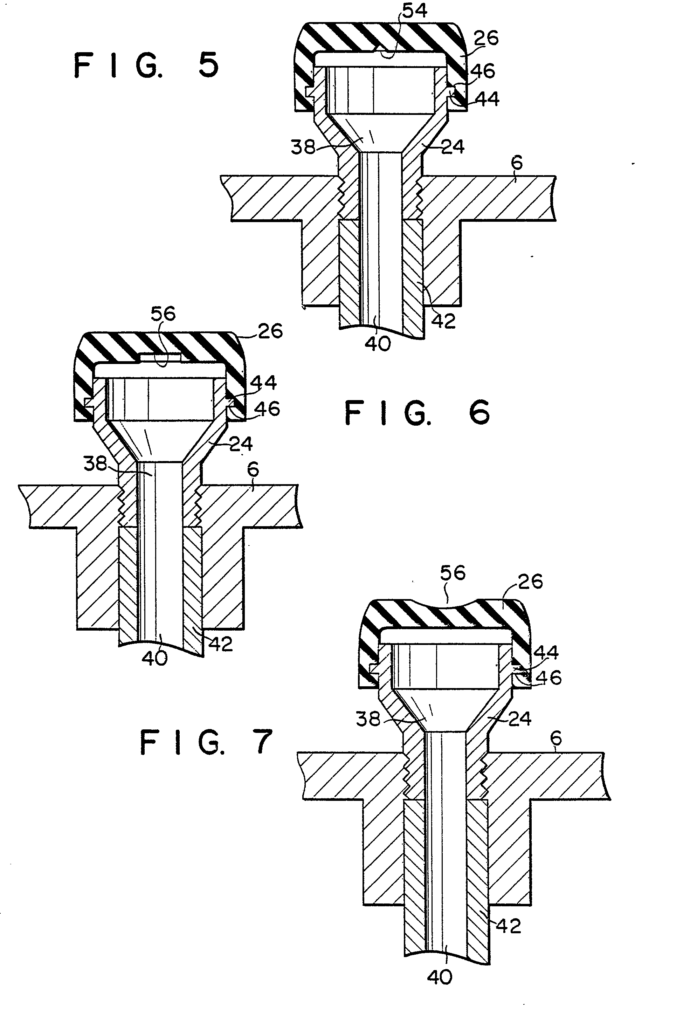

According to the above-described example, the plug 26 is made thinner in such a way that the conical recess 48 is formed in the center portion of upper surface thereof, but the present invention is not limited to this example and the plug 26 may be made thinner as shown in Figs. 5 through 7. Namely, the conical recess 54 may be formed in the center portion of inner surface of plug 26 as shown in Fig. 5. Or a cylindrical recess 56 having a relatively large area may be formed in the center portion of inner surface of plug 26 as shown in Fig. 6. Or a recess 56 of circular arc having a relatively large area may be formed in the center portion of upper surface of plug 26 as shown in Fig. 7. Same parts in Figs. 5 through 7 as those in the first example are represented by same reference numerals and description on these parts will be omitted.

As apparent from the above, the cap-shaped plug is fitted onto the outer circumference of holder, thus making it easy to detachably attach the plug to the holder. In addition, the thinner portion of plug enables operating tools such as forceps to easily penetrate through the plug into the forceps inlet. Further, the plug is made of resilient material, so that the through-hole formed in the thinner portion of plug is closed by the restoring force of resilient plug itself, thus preventing liquid and the like in the body cavity from leaking outside the forceps inlet through the forceps channel.

- 1 -

0065187

Claims:

1. A plug for closing the forceps inlet of an endoscope characterized by comprising:

a first portion to be freely detachably attached to a member (24) which defines the forceps inlet (38) and having a shape corresponding to that of said member (24); and

a second portion for closing the forceps inlet (38) and having a thinner area (48, 54, 56) through which an operating tool (50) is to be penetrated, said first and second portions being made integral of resilient material.

2. A plug according to claim 1, characterized in that said second portion has a flat inner surface and an outer surface in which a recess (48, 56) is formed.

3. A plug according to claim 1, characterized in that said second portion has a flat outer surface and an inner surface in which a recess (54, 56) is formed.

4. A plug according to claims 1 to 3, characterized in that said plug is cap-shaped.

5. A plug according to claim 1, characterized in that said second portion has a recess (46) which is engaged with a projection (44) formed on said member (24).

# F I G. 1

F I G. 2

F I G. 3

F I G. 4

F I G. 5

F I G. 6

F I G. 7